Europäisches Patentamt

European Patent Office

Office européen des brevets

(19)

(11) Veröffentlichungsnummer: **0 284 074**
**A1**

# (12) EUROPÄISCHE PATENTANMELDUNG

(21) Anmeldenummer: 88104763.3

(22) Anmeldetag: 24.03.88

(51) Int. Cl.4: **C01B 25/32** , //A61L27/00

(30) Priorität: 26.03.87 DE 3709897

(43) Veröffentlichungstag der Anmeldung:
28.09.88 Patentblatt 88/39

(84) Benannte Vertragsstaaten:
AT BE CH DE ES FR GB GR IT LI LU NL SE

(71) Anmelder: **Ewers, Rolf, Prof. Dr. Dr.**
**Graf-Spee-Strasse 46**
**D-2300 Kiel 1(DE)**

(72) Erfinder: **Ewers, Rolf, Prof. Dr. Dr.**
**Graf-Spee-Strasse 46**
**D-2300 Kiel(DE)**
Erfinder: **Simons, Bruno, Dr.**
**Tauernweg 42**
**D-2300 Kiel(DE)**
Erfinder: **Kasperk, Christian, Dr.**
**Lorenzendamm 20**
**D-2300 Kiel(DE)**

(74) Vertreter: **Weber, Otto Ernst, Dipl.-Phys. et al**
**Hofbrunnstrasse 36**
**D-8000 München 71(DE)**

(54) Verfahren zur Herstellung eines Hydroxylapatitmaterials.

(57) Es wird ein Verfahren zur Herstellung eines Hydroxylapatitmaterials mittels hydrothermaler Umsetzung beschrieben, wobei als Ausgangsstoff ein von organischen Substanzen gereinigtes calcitisches Skelett dient. Die Umsetzung erfolgt unter Sättungsdampfdruck bevorzugt in einem Temperaturbereich zwischen 100° C und 250° C. Die Beeinflussung der Reaktion bzw. der Defektkonzentration des Materials erfolgt bevorzugt durch Zugabe von $F^-$ Ionen. Ferner wird die Adaption des Materials an wechselnde Einsatzbereiche als Hartgewebsersatz sowie die Herstellung bearbeitbarer kompakter Implantate beschrieben.

EP 0 284 074 A1

Xerox Copy Centre

## Verfahren zur Herstellung eines Hydroxylapatitmaterials

Die Erfindung betrifft ein Verfahren zur Herstellung eines carbonatischen Hydroxylapatitmaterials mittels hydrothermaler Umsetzung unter Zugabe einer wässrigen $(NH_4)_2HPO_4$-Lösung, wobei als Ausgangsstoff ein von organischen Substanzen gereinigtes Hartgewebe dient.

Ein derartiges Hydroxylapatitmaterials ist als biokompatibles Implantat, beispielsweise als Hartgewebe-Ersatz für Knochen und Zähne, geeignet, da der anorganische Teil des menschlichen Hartgewebes aus einem kryptokristallinen carbonatischen Hydroxylapatit besteht.

Aus der U.S. PS 3,929,971 ist ein Verfahren bekannt, mit welchem aus dem aragonitischen Skelett von rezenten Korallen und Seesternen durch hydrothermalen Umsatz ein Hydroxylapatitmaterial gewonnen werden kann. Dieses bekannte Verfahren hat den Nachteil, daß die hydrothermale Umsetzung bei einer relativ hohen Temperatur unter einem hohen Druck ausgeführt werden muß.

Im Hinblick auf eine Verwendung des Hydroxylapatitmaterials als Implantat ist nachteilig, daß bei den bekannten Herstellungsverfahren als Funktion der Temperatur, des Druckes und der chemischen Potentiale der beteiligten Komponenten keine hohen Defektkonzentrationen auftreten, die eine knochengleiche Abweichung des Materials vom Idealbau eines dichten Materials des Chemismuses $Ca_{10}(PO_4)_6(OH)_2$ mit Apatitstruktur bewirken. Diese Defektkonzentrationen können beispielsweise durch die Substitution von $CO_3^{2-}$ für $PO_4^{3-}$ oder von Fremddionen, vor allem Alkalien im Kationteilgitter, sowie von Halogeniden im anionteilgitter auftreten. Ferner erfüllt das mit Hilfe des bekannten Verfahrens hergestellte Produkt nicht die gewünschten Anforderungen an die knochenähnliche Kryptokristallinität im Hinblick auf die Verwendung als Implantat.

Der Erfindung liegt die Aufgabe zugrunde, ein Verfahren der eingangs genannten Art anzugeben, welches bei geringerem Energiebedarf und relativ niederem Druck in kurzer Zeit ausgeführt werden kann und die gewünschte knochenähnliche Defektkonzentration und Kryptokristallinität einstellt.

Diese Aufgabe wird dadurch gelöst, daß die hydrothermale Umsetzung in einem Autoklav bei Sättigungsdampfdruck der jeweiligen flüssigen Phase vorgenommen wird. Der Sättigungsdampfdruck stellt sich ein, wenn das Druckgefäß nicht vollständig gefüllt wird. Es kann bereits bei Zeiten, die kürzer als 24 Stunden sind, und einer Temperatur von 200°C ein optimaler Umsatz erreicht werden.

Eine bevorzugte Weiterbildung der Erfindung besteht darin, daß über eine kontrollierte Einstellung des pH-Wertes der Substitutionslösung, vorzugsweise auf mindestens 8,5, die Bildung anderer Phasen als Hydroxylapatit unterdrückt wird.

Des weiteren kann es vorteilhaft sein, daß der Einfluß von $Mg^{2+}$-Ionen durch entsprechende Zugabe von F-Ionen unterdrückt wird. Diese Maßnahme hat den Vorteil, daß der Gehalt von $Mg^+$-Ionen kompensiert und damit die Bildung unerwünschten $\beta$-Tricalciumphosphats ($\beta$-Whitlockit) verhindert wird. Ferner wird die Kristallinität und Defekt konzentration des Materials, d. h. der Realbau des Materials und damit die Adaption des Materials an den Einsatzbereich, durch die Zugabe eines Fluorids während der Synthese erreicht. Dies führt zu einer künstlichen Alterung bzw. Härtung des Materials.

Bevorzugt wird das Verfahren dadurch weitergebildet, daß zur Nachbehandlung des Hydroxylapatitmaterials eine Temperung erfolgt. Auch durch diese Maßnahme kann die künstliche Alterung oder Härtung des Materials erreicht werden. Über die Temperatur wird die Defektkonzentration beeinflußt und über die Temperzeit die Kristallitgröße gesteigert.

Insbesondere zur Herstellung eines kompakten Implantats ist es vorteilhaft, daß das Hydroxylapatitmaterial mit einem Bindemittel eingeschlämmt und die zur hydrothermalen Umwandlung vorgenommenen Verfahrensschritte wiederholt werden. Das auf diese Weise erhaltene Material kann mit herkömmlichen Werkzeugen bearbeitet werden.

Als Alternative kann es vorteilhaft sein, daß das Ausgangsmaterial vor der hydrothermalen Umsetzung mit einem Bindemittel eingeschlämmt wird und die Umsetzung des Ausgangsmaterials und des Bindemittels gleichzeitig erfolgt. Auf diese Weise kann der Arbeitsprozess beschleunigt werden.

Bevorzugt erfolgt das Einschlämmen dadurch, daß als Bindemittel gelöschter Kalk verwendet wird und die hydrothermale Umwandlung nach dem Abbinden des Kalkes erfolgt. Zur Formgebung ist es zweckmäßig, daß das Hydroxylapatitmaterial in einen Formkörper eingerüttelt wird.

Besonders vorteilhaft ist es, daß das Hydroxylapatitmaterial vor dem Einschlämmen in den Formkörper eingerüttelt wird.

Es erweist sich auch als sehr vorteilhaft, daß als Ausgangsstoff das Skelett von kalkinkrustierenden Algen verwendet wird. Dieses Skelett kann auch decarbonatisiert werden. Damit läßt sich ein Hydroxylapatitmaterial erzeugen, welches eine äußerst feine Oberfläche, Porosität und zythophile Oberflächen-Geometrie aufweist und organogenem Material äußerst ähnlich ist. Durch die erfindungsgemäße Synthese bleibt die vorgegebene interkonnektierende Mikroporosität des Ausgangsmaterials erhalten. In der klinischen Erprobung hat es sich erwiesen, daß der hierbei erhaltene Hydroxylapatit sowohl biokompatibel als auch bioaktiv ist, und daß er sowohl in den Knochenbildungsprozeß integiert wird als auch aktiv die Osteogenese initiiert und unterstützt.

Alternativ dazu ist als Ausgangsstoff auch eine organogenes, poröses Hartgewebe sehr gut geeignet. Es kann zweckmäßig sein, daß das Hartgewebe vor der Umsetzung decarbonatisiert wird.

Mit der Erfindung wird ein Verfahren aufgezeigt, welches insbesondere im Temperaturbereich bis 250° C, und einem Druck bis 40 bar einen effizienten Umsatz bei geringen Material-und Energiekosten gewährleistet. Es zeichnet sich vor allem gegenüber Hydrothermal-Synthesen aus, die eine externe Druckerzeugung erfordern und bei hohen Temperaturen Edelmetallbehälter benötigen. Außerdem können Knochenersatzmaterialien erzeugt werden, die knochenähnlich sind und sich hinsichtlich ihrer Kristallit größen und ihrer Defektkonzentrationen ausgezeichnet für Implantate eignen.

Nachfolgend wird das Verfahren anhand eines Ausführungsbeispiels weiter beschrieben.

Zunächst werden die organischen Bestandteile des Ausgangsstoffes vollständig entfernt. Dabei kann der Ausgangsstoff auch bei höherer Temperatur decarbonatisiert werden. Daran schließen sich die folgenden Verfahrensschritte an:

1. Säuberung des Ausgangsstoffes

1.1 Separierung grobkörniger Fremdpartikel unter dem Binokular

1.2 Mehrmaliges Auswaschen des Ausgangsstoffes mit aqua bidestillata, gegebenenfalls bei erhöhter Temperatur

2. Röntgenographische Bestimmung des $Mg^{2+}$-Gehaltes des Ausgangsstoffes und Zugabe von $F^-$ Ionen zur Kompensation des $Mg^+$-Gehaltes. Diese Kompensation kann durch Zugabe von $NH_4F$, Alkali-Fluoriden, $CAF_2$, HF oder ähnlichem erfolgen.

3. Synthese
Der Ausgangsstoff wird in einen mit dauerthermoplastischem Polytetrafluoräthylen (PTFE) ausgekleideten Autoklav eingebracht und mit einer konzentrierten, wässrigen Lösung von $(NH_4)_2HPO_4$ überschichtet. Gegebenenfalls wird zur Kompensation des $Mg^+$-Gehaltes ein entsprechendes Fluorid zugesetzt, wie in Abschnitt 2 bereits beschrieben wurde. Der pH-Wert wird auf ca. 8,5 eingestellt. Der Autoklav ist nicht vollständig gefüllt, so daß bei erhöhter Temperatur der Sättigungsdampfdruck sich einstellt. Die Maximaltemperatur ist durch die Auskleidung des Autoklavs mit ca. 250°C vorgegeben. Bereits bei ca. 200°C kann bei Zeiten, die kürzer als 24 Stunden sind, ein optimaler Umsatz erreicht werden.

4. Nachbehandlung des Materials
Nach der Reaktion wird der pH-Wert kontrolliert - der unverändert sein soll - und die wässerige Phase verworfen. Das umgesetzte Hydroxylapatitmaterial wird mehrmals mit aqua bidestillata gewaschen und gegebenenfalls kurz aufgekocht. Anschließend wird das Ausgangsmaterial bei einer Temperatur größer 100°C getrocknet.

5. Künstliche Alterung bzw. Härtung des Hydroxylapatitmaterials
Die Kristallinität und Defektkonzentration des Materials, d. h. der Realbau des Materials und damit die Adaption des Materials an dem Einsatzbereich, wird entweder durch Zugabe eines Fluorids in der bereits beschriebenen Weise während der Synthese erreicht, oder durch Temperung des Materials bei Temperaturen bis etwa 500°C. Damit wird einerseits über die Temperatur der "Karbonat-Gehalt" der Probe beeinflußt und über die Temperzeit die Kristallitgröße gesteigert.

6. Kompaktierung des Hydroxylapatitmaterials
Das umgesetzte Ausgangsmaterial wird in einen Formkörper eingerüttelt und mit gelöschtem Kalk $(Ca(OH)_2)$ eingeschlämmt. Nach dem Abbinden des Kalkes wird der Formkörper. inklusive Inhalt wiederum dem im Abschnitt 3 beschriebenen Syntheseverfahren unterworfen. Dabei wird auch das kalkige Bindemittel in Hydroxylapatit umgesetzt.

Es ist prinzipiell möglich, den carbonatischen oder oxidischen Ausgangsstoff direkt einzuschlämmen und in einem Arbeitsgang das carbonatische oder oxidische Skelett und das Bindemittel zu Hydroxylapatit umzusetzen. Eine künstliche Alterung kann entsprechend Abschnitt 5 erfolgen.

Durch entsprechende Variation des Druckes, der Temperatur und der chemischen Potentiale ist es auch möglich, Whitlockit ($\beta$-$Ca_3(PO_4)_2$) herzustellen.

Sehr gute Ergebnisse können mit folgenden Verfahrensschritten erzielt werden:

1. Trocknen des Ausgangsmaterials, das calcitischen oder aragonitischen Ursprungs sein kann.

2. Pyrolyse des Materials: Lineares Aufheizen über 6 h auf 750°C, d.h. auf eine Temperatur, die oberhalb der Zersetzungstemperatur des Ausgangsmaterials liegt; Halten bei dieser Temperatur über 6 h; lineares Abkühlen über 6 h. Verfahrenstechnisch besonders wesentlich ist hierbei das allmähliche Aufheizen. Dieser Pyrolyse-Schritt beeinflußt die Reaktion und das Produkt durch Auflockerung der kristallinen Packung positiv.

3. Fraktionierung des Materials durch eine Prüfsieb-Kolonne.

4. Ein mit PTFE-ausgekleideter herkömmlicher Autoklav mit 400 ml Volumen wird bis zur Hälfte mit dem aus den vorgehenden Verfahrensschritten erhaltenen Material gefüllt. Eine vorbereitete Lösung von 68 g $(NH_4)_2HPO_4$ und 1 g $NH_4F$ pro 100 ml $H_2O$ wird bis zu einer Gesamtfüllung des Autoklaven von 75 % zugesetzt. Das Volumen des Autoklavs ist also so groß bemessen, daß die konzentrierte phosphatische Lösung in einem Überschuß zugegeben werden kann, der eine ausreichende Sättigung während der gesamten Reaktion gewährleistet. Der Überschuß kann empirisch leicht ermittelt werden.

5. Der Autoklav wird in einen Trockenschrank gelegt und verbleibt dort zwischen 8 und 16 h bei 200° C.

6. Der Autoklav wird dann in Wasser zum Abkühlen gestellt.

7. Der gesamte Autoklav - Inhalt wird in einen Erlenmeyer-Kolben überführt und mit reichlich $H_2O$ merhfach ausgespült; danach wird zweimal mit reichlich $H_2O$ aufgekocht.

8. Erste Trocknung unter Infrarotlicht; dann weitere Trocknung bei 110° C im Trockenschrank und anschließend ca. 4 h bei 200° C, was einer gleichzeitigen Trockensterilisation gleichkommt.

, Als weiterer Verfahrensschritt ist es vorteilhaft, das Material vor der Umsetzung zu dekarbonatisieren. Bei der Entgasung werden die Porenräume aufgerissen und somit die Oberfläche vergrößert.

Es ist besonders wichtig, daß die phosphatische Lösung konzentriert ist, daß der pH-Wert im basischen Bereich liegt, bevorzugt bei mindestens 8,5, und die Lösung in einem Überschuß in Relation zum Material zugegeben wird, so daß die Konzentration der Lösung während der Reaktion nicht unter ein vorgegebenes Limit fällt und der pH-Wert in dem vorgegebenen gewünschten Bereich bleibt. Auf diese Weise wird eine vollständige Umwandlung zu Hydroxylapatit erreicht, ohne daß dabei Spuren von Whitlockit erzeugt werden. Bei Mg-haltigen calci tischen Skeletten als Ausgangsmaterial wird durch Zugabe von $NH_4F$ von mindestens 1 g pro 100 ml phosphatischer Lösung eine Umwandlung zu Whitlockit verhindert. Ein auf diese Weise erhaltener carbonatischer Hydroxylapatit kann Vorteile für die klinische Applikation zeigen, beispielsweise eine geringere Resorbierbarkeit.

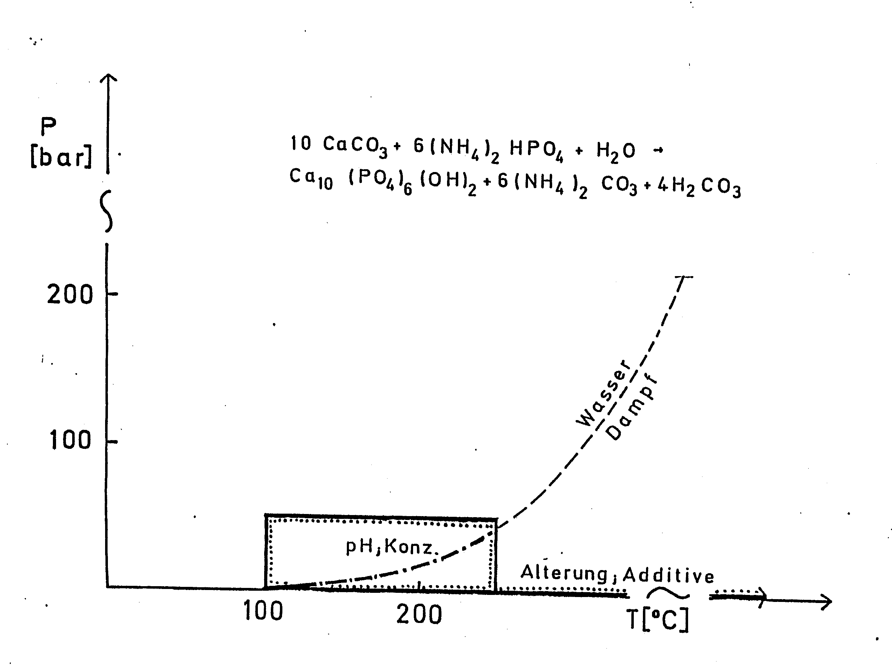

Wichtige Verfahrensparameter sind in der Figur wiedergegeben.

Der an sich bekannte Autoklav besteht aus einem verschließbaren, auf hohen Überdruck geprüften Metallgefäß mit aufgeschraubtem oder aufgepreßtem dicht schließenden Deckel, in dem sich ein verstellbares Sicherheitsventil, ein Manometer und ein Thermometer befinden können. Am Boden des Autoklavs befindet sich Wasser, das von außen erhitzt wird. Der Dampfdruck steigt dann im Innern bis zu dem am Ventil angezeigten Wert an. Hernach wird das Ventil gehoben und der überschüssige Dampf entweicht.

Zusammenfassend zeichnet sich die Erfindung dadurch aus, daß wegen der niedrigen Temperaturen und der selbständigen Druckerzeugung unter geringstem Einsatz von energie Hydroxylapatit hergestellt werden kann. Die unerwünschte Bildung von anderen Phosphaten, wie vor allem Whitlockit, wird durch die korrekte Einstellung und Beibehaltung eines basischen pH-Wertes vermieden. Durch die vorgegebene ausreichende Konzentration der Phosphat-Ionen in der fluiden Phase während der gesamten Reaktionszeit wird ein vollständiger Umsatz erreicht. Ferner wird durch die Zugabe von $F^-$-Ionen die Gegenwart von Mg-Ionen kompensiert und somit wiederum die Bildung unerwünschter Phasen vermieden. Durch die erfindungsgemäße Pyrolyse des Materials bleibt die Mikroporosität erhalten. Die spezifische Oberfläche wird durch Decarbonatisierung sowie die Fluorisierung, Kompaktierung und Beeinflussung des Carbonatgehalts erhöht. Eine externe Druckerzeugung ist nicht erforderlich. Es kann auf eine Mazeration des Materials verzichtet werden.

**Ansprüche**

1. Verfahren zur Herstellung eines carbonatischen Hydroxylapatitmaterials mittels hydrothermaler Umsetzung unter Zugabe einer wässerigen $(NH_4)_2HPO_4$-Lösung, wobei als Ausgangsstoff ein von organischen Substanzen gereinigtes Hartgewebe dient,
dadurch **gekennzeichnet**,
daß die Umsetzung in einem Autoklav bei Sättigungsdampfdruck der jeweiligen flüssigen Phase vorgenommen wird.

2. Verfahren nach Anspruch 1,
dadurch **gekennzeichnet**,
daß der pH-Wert der Substitutionslösung definiert eingestellt wird.

3. Verfahren nach Anspruch 2,
dadurch **gekennzeichnet**,
daß der pH-Wert auf mindestens 8,5 eingestellt wird.

4. Verfahren nach einem der vorhergehenden Ansprüche 1 bis 3,
dadurch **gekennzeichnet**,
daß eine Kompensation von $Mg^{2+}$-Ionen durch entsprechende Zugabe von $F^-$-Ionen vorgenommen wird.

5. Verfahren nach einem der vorhergehenden Ansprüche,
dadurch **gekennzeichnet**,
daß zur Nachbehandlung des Hydroxylapatitmaterials eine Temperung erfolgt.

6. Verfahren nach einem der vorhergehenden Ansprüche,
dadurch **gekennzeichnet**,
daß das Hydroxylapatitmaterial mit einem Bindemittel eingeschlämmt und die zur hydrothermalen Umwandlung vorgenommenen Verfahrensschritte wiederholt werden.

7. Verfahren nach Anspruch 1,
dadurch **gekennzeichnet**,
daß das Ausgangsmaterial vor der hydrothermalen Umsetzung mit einem Bindemittel eingeschlämmt wird und die Umsetzung des Ausgangsmaterials und des Bindemittels gleichzeitig erfolgt.

8. Verfahren nach einem der vorhergehenden Ansprüche,
dadurch **gekennzeichnet**,
daß als Bindemittel gelöschter Kalk verwendet wird und die hydrothermale Umwandlung nach dem Abbinden des Kalkes erfolgt.

9. Verfahren nach einem der vorhergehenden Ansprüche,
dadurch **gekennzeichnet**,
daß das Hydroxylapatitmaterial in einen Formkörper eingerüttelt wird.

10. Verfahren nach Anspruch 9,
dadurch **gekennzeichnet**,
daß das Hydroxylapatitmaterial vor dem Einschlämmen in den Formkörper eingerüttelt wird.

11. Verfahren nach einem der vorhergehenden Ansprüche,
dadurch **gekennzeichnet**,
daß als Ausgangsstoff das Skelett von kalkinkrustierenden Algen verwendet wird.

12. Verfahren nach einem der Ansprüche 1 bis 10,
dadurch **gekennzeichnet**,
daß als Ausgangsstoff ein organogenes, poröses Hartgewebe verwendet wird.

13. Verfahren nach einem der vorhergehenden Ansprüche,
dadurch **gekennzeichnet**,
daß das Hartgewebe vor der Umsetzung decarbonatisiert wird.

$$10\ CaCO_3 + 6\ (NH_4)_2\ HPO_4 + H_2O \rightarrow$$
$$Ca_{10}\ (PO_4)_6\ (OH)_2 + 6\ (NH_4)_2\ CO_3 + 4\ H_2CO_3$$

pH, Konz.

Alterung, Additive

Wasser / Dampf

P [bar]

200

100

100   200   T [°C]

0 284 074

| | **EINSCHLÄGIGE DOKUMENTE** | | EP 88104763.3 | |
|---|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) | |
| D,X | US - A - 3 929 971 (DELLA M. ROY)<br>* Ansprüche * | 1,5-13 | C 01 B 25/32<br>A 61 L 27/00 | |
| P,X | DE - C1 - 3 542 744 (EWERS)<br>* Ansprüche; Spalte 2, Zeilen 30-59 * | 1,5-13 | | |

RECHERCHIERTE
SACHGEBIETE (Int. Cl.4)

C 01 B 25/00

A 61 L

C 01 D

Der vorliegende Recherchenbericht wurde für alle Patentansprüche erstellt.

| Recherchenort | Abschlußdatum der Recherche | Prüfer |
|---|---|---|
| WIEN | 24-06-1988 | PAMMINGER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, überein- stimmendes Dokument

EPA Form 1503 03 82